# EUROPEAN PATENT APPLICATION

(11) **EP 1 555 031 A1**
(43) Date of publication of application: **20.07.2005**
(21) Application number: 03809416.5
(22) Date of filing: 28.03.2003
(51) Int. Cl.: A61K 38/17, A61K 9/19, A61K 35/12, A61K 47/36, A61L 27/22, A61L 27/38, A61P 41/00

(54) **ANGIOGENESIS INDUCER**

(30) Priority: 23.10.2002 JP 2002308117
(71) Applicant: Inoue, Kazutomo, Kyoto-shi, Kyoto 606-8163 (JP); Creative Co., Ltd., Kyoto-shi, Kyoto 600-8095 (JP)
(72) Inventor: INOUE, Kazutomo, Sakyo-ku, Kyoto 606-8163 (JP); GU, Yuanjun, Kyoto-shi, Kyoto 606-8507 (JP); KIM, Dohoon, Kyoto-shi, Kyoto 606-8507 (JP)
(74) Representative: Teipel, Stephan, Dr.
(86) International application number: PCT/JP2003/004052
(87) International publication number: WO 2004/037280

(57) **Abstract**

It is intended to provide an angiogenesis inducer comprising fibrin which is safe to a living body. The angiogenesis inducer is characterized by being capable of safely inducing angiogenesis when administered to a living body, which makes it possible to achieve functional regeneration of living tissues or organs suffering from dysfunction or malfunction.

## Description

### TECHNICAL FIELD

The present invention relates to an angiogenesis inducer useful as a pharmaceutical composition.

### BACKGROUND ART

Angiogenesis is the formation of new small blood vessels from existing blood vessels, and many researchers have vigorously studied the mechanism of angiogenesis. Angiogenesis is involved in development or metastasis of cancer, and progression of diabetic retinopathy and inflammatory disease (chronic rheumatoid arthritis), and there are many studies aiming at suppression of angiogenesis for cancer treatment. On the other hand, in recent years, an innovative new therapy called "angiogenesis therapy" for protecting ischemic tissues and treating an affected part by utilizing and promoting this angiogenesis action to actively supply sufficient blood to surrounding area of the ischemic tissues has been studied.

A drug therapy for an ischemic disease has insufficient effect of improving ischemia in many cases and, for those ischemic diseases that are nonresponsive to a drug therapy, circulation reconstruction is not possible due to complications such as cerebrovascular disease and renal dysfunction. In addition, there is no effective therapy for a circulatory disease such as peripheral angiopathy typically, for example, arteriosclerosis obliterans and Buerger disease and, when vasodilation and surgical anagioplasty are difficult, amputation of the lower extremity is necessary. As a new therapy for such serious ischemic disease cases and circulation diseases, angiogenesis therapy for promoting angiogenesis to form new blood vessels is effective. However, these techniques have not been put into practice yet, and development of an excellent drug which can effectively and safely induce angiogenesis is expected.

As one specific example of development of angiogenesis therapy, there is a study of an angiogenesis technique utilizing embryonic stem cells (ES cell) (e.g. Hirashima M., Kataoka H. et al., "maturation of embryonic stem cells into endothelial cells in an in vitro model of vasculogenesis" , Blood, American Society of Hematology (U.S.A), February 15 1999, vol. 93, No. 4, p.1253-1263), but necessary techniques to be established, such as a culturing method, a differentiation inducing method and a method of obtaining differentiated cells are not completed, and the above angiogenesis technique has not been practically used.

In addition, similarly as the above, implantation of self bone-marrow cells for angiogenesis by directly introducing bone-marrow mononuclear cells separated from human bone-marrow fluid into a site to be treated (e.g. Jubun Shimada and Toyoaki Murohara, "Therapeutic Angiogenesis by Transplantation of Self Bone-marrow Cells", Regenerative Medicine/Regenerative therapy, Chemistry Today, supplement, TOKYO KAGAKU DOZIN Co., Ltd. July 1, 2002, vol.41, p.102-108) has been already studied. However, also in this method, it is necessary to collect a large amount of bone-marrow fluid by subjecting a patient to systemic anesthesia, and thus physical burden and risks of a patient can not be avoided. Further, there is a problem that it is remarkably difficult to control differentiation of introduced cells.

Therefore, a technique which can induce angiogenesis in vivo by introducing fibrin, which is used as a hemostat in surgical operation and is safe to a living body, into a living body as the method according to the present invention has not yet been known at all. In addition, the present invention is safer in that a growth factor having a possibility of side effect by administration due to insufficient clarification of its mechanism is not necessarily used, and that surgical burden such as collection of bone-marrow fluid is not imposed on a patient. Furthermore, fibrin is excellent in adherability to a living body, and can be easily fixed at a target site such as an affected part when administered to a living body.

Therefore, by utilizing the angiogenesis induction according to the present invention, angiogenesis therapy for treatment of diseases by forming blood vessels around an affected part to supply sufficient blood flow, which is clinically and economically practical, can be realized.

### DISCLOSURE OF INVENTION

An object of the present invention is to provide an angiogenesis inducer which is safe to a living body. More specifically, an object of the present invention is to provide an angiogenesis inducer comprising fibrin which induces angiogenesis by administering fibrin which is safe to a living body and is excellent in adherability to a living body to an organ or tissues, and a further object of the present invention is to provide an angiogenesis inducer which is desired in the field of regenerative medicine.

The present inventors conducted intensive studies, and found an entirely unexpected and novel finding that administration of fibrin to a living body induces angiogenesis. In addition, the present inventors found that by administration of fibrin to a living body, which induces angiogenesis, oxygen and nutrients necessary for proliferation and maintenance of cells can be supplied, and as a result, functional regeneration of living tissues and organs suffering from dysfunction or malfunction can be achieved.

Based on these findings, the present inventors continued to study and, as a result, completed the present invention.

That is, the present invention relates to:
(1) an angiogenesis inducer comprising fibrin,
(2) the angiogenesis inducer according to (1), further comprising a biodegradable polymer,
(3) the angiogenesis inducer according to (1), further comprising a cell selected from the group consisting of bone-marrow mononuclear cell, bone-marrow stromal cell, stem cell, keratinocyte, fibroblast, myocardial cell, neural stem cell, vascular endothelial cell, endothelial progenitor cell, vascular epithelial cell, osteoblast, chondrocyte, smooth muscle cell, skeletal muscle cell, pancreatic cell, renal cell, enterocyte and stomach cell and/or a tissue comprising said selected cell,
(4) the angiogenesis inducer according to (1), further comprising a growth factor,
(5) a method of inducing angiogenesis, comprising treating a living body with fibrin,
(6) a granule preparation produced by freeze-drying fibrin obtained by enzymatic degradation of fibrinogen,
(7) a granule preparation produced by freeze-drying a mixture of fibrin obtained by enzymatic degradation of fibrinogen and calcium,
(8) a skin graft method comprising using fibrin,
(9) a method for prevention or treatment of skin disease, comprising using fibrin,
(10) a method for prevention or treatment of peripheral vascular disease, comprising using fibrin,
(11) a method for prevention or treatment of cardiac disease comprising using fibrin,
(12) a method for prevention or treatment of brain disease comprising using fibrin,
(13) a method for prevention or treatment of bone disease comprising using fibrin,
(14) a method for subcutaneously transplanting an artificial organ comprising using fibrin,
(15) a method for prevention or treatment of respiratory disease comprising using fibrin,
(16) a method for prevention or treatment of digestive organ disease, comprising using fibrin,
(17) a method for prevention or treatment of endocrine and metabolism disease comprising using fibrin,
(18) a method for prevention or treatment of autoimmune disease, and
(19) use of fibrin for inducing angiogenesis.

### BRIEF DESCRIPTION OF THE DRAWINGS

Fig. 1 is a view showing the take state of a skin flap 3 days and 7 days after formation of the skin flap in a group of administration of fibrin prepared in Example 1 and a control group.
Fig. 2 is a view showing a skin flap take rate (%)on day 3 (a) and (b) and day 7 (c) and (d) after formation of the skin flap in a group of administration of fibrin prepared in Example 1 and a control group.
Fig. 3(a) and (b) show HE staining of a tissue obtained by collecting each skin flap on day 7 after formation of the skin flap in a group of administration of fibrin prepared in Example 1 (a) and a control group (b). Fig.3(c) shows HE staining of a tissue obtained by collecting a skin flap on day 50 after formation of the skin flap in a group of administration of fibrin prepared in Example 1.
Fig. 4 shows the time course measurements of blood flow rate (ml/100 g tissue/min) of each subcutaneous tissue on day 3 (a) and (b) and on day 7 (c) and (d) after formation of the skin flap in a group of administration of fibrin prepared in Example 1 and a control group.
Fig. 5 shows a recovery rate (%) of each of blood flow rate of a subcutaneous tissue at 0.5 cm position (a) and 1.5 cm position (b) on a skin flap on day 1, day 3 and day 7 after formation of the skin flap in a group of administration of fibrin prepared in Example 1 and a control group.
Fig. 6 shows a recovery rate (%) of each of a skin flap epidermal temperature on day 3 and day 7 after formation of the skin flap in a group of administration of fibrin prepared in Example 1 and a control group.
Fig. 7 shows the time course changes in blood flow rate (ml/100 g tissue/min) at an ischemic site of a rat ischemia model with a femoral artery cut 5 day after administration of fibrin prepared in Example 1 to the site.

### BEST MODE FOR CARRYING OUT THE INVENTION

Fibrin used in the present invention is not particularly limited, but it may be a commercially available fibrin powder, a preparation produced from a commercially available fibrinogen, or a preparation produced from fibrinogen obtained by purification of human or animal plasma. Alternatively, the fibrin may be prepared from a fibrinogen-containing cell culture obtained by a recombinant DNA technique for fibrinogen production. In addition, it is desirable that those fibrins are freeze-dried fine granule in a form that can be easily suspended in a solution such as physiological saline, phosphate buffer or the like. Examples of such commercially available fibrin include a dried fibrin for medical use prepared according to Guideline for Biological Product(1979, p.2001-2003) supervised by Ministry of Health, Labor and Welfare, Pharmaceutical Affairs Bureau, but preferably such fibrins are those having been clinically put into practice as a hematostat from which a virus is removed.

A subject to which the angiogenesis inducer of the present invention is administered includes a human and other mammal.

When fibrinogen is isolated from human or animal plasma, it is desirable to isolate fibrinogen from human plasma when a human is the subject, and from animal plasma when an animal is the subject, in light of biocompatibility. A method of isolating fibrinogen is not particularly limited, and the known plasma fractionation method may be used. Further purification of a fibrinogen precipitate obtained by the plasma fractionation may be performed by the technique well-known to a person skilled in the art, for example, reprecipitation of fibrinogen using a protein precipitate in the presence of a salt and/or amino acid, or chromatography technique (e.g. ion exchange, affinity, hydrophobic or gel permeation chromatography), or a combination of both techniques. It is preferable that a plasma-polluting substances mixed therein are removed. For example, it is preferable that fibronectin and plasminogen are absorbed and removed by immobilized gelatin and immobilized lysine, respectively. Fibrin prepared from fibrinogen which has been subjected to the above procedure has suppressed autolysis, and is stable for a long term.

In addition, it is preferable that fibrinogen prepared from human or animal plasma is subjected to thermal treatment, chemical treatment or the like to remove viruses harmful to a living body.

Isolated fibrinogen may be suspended in a suitable aqueous solvent or the like to prepare a fibrinogen solution. Alternatively, this fibrinogen solution may be freeze-dried to produce a freeze-dried fibrinogen product. A freeze-drying method may be according to the known per se technique.

When fibrin is prepared from commercially available fibrinogen, or fibrinogen isolated from human or animal plasma, it is preferable to prepare fibrin using high purity thrombin from which viruses have been removed. As high purity thrombin, commercially available products listed in Pharmacopoeia such as an oral thrombin fine granule which is used as an organ hemostat are preferable, and thrombin which has usually biological activity or physiological activity as thrombin, for example, thrombin obtained by fractionating a plasma protein may be used. That is, for example, thrombin prepared by acting thromboplastin or snake venom on prothrombin purified from human or bovine plasma in the presence of Ca²⁺, followed by purification, may be used. Purification of thrombin is preferably performed by hydrophobic interacting chromatography (HIC) alone or in combination with cation exchange chromatography (CEC).

In the aforementioned preparation of fibrin, the concentration of fibrinogen at the time of dissolution is 4 to 12 w/v%, preferably 6 to 10 w/v%, because an adhesion strength of the prepared fibrin is enhanced in said concentration region. As a solvent for dissolving fibrinogen, an aqueous solvent such as distilled water for injection, physiological saline for injection, and a buffer (phosphate series, citric acid series etc.) having a pH of 5 to 18 can be used.

In order to form stable fibrin without lowering a crosslinking degree (polymerization degree) of fibrin, the amount of thrombin to be added to the aforementioned fibrinogen solution is preferably 0.07 to 0.36 unit of thrombin, more preferably 0.07 to 0.25 unit of thrombin per 1 mg of fibrinogen. With regard to the unit of thrombin, an amount usually required for coagulating 1mn of a 0.1% purified fibrinogen solution in 15 seconds is taken as 1 unit (NIH (National Institute of Health in the U.S.) unit): Minimum Requirements for Dried Thrombin (1946)).

In the present invention, it is preferable that commercially available fibrinogen, or fibrinogen prepared from human or animal (e.g. bovine) plasma is suspended in the aforementioned solvent, an appropriate amount of thrombin is added thereto, and then incubated at around 37°C overnight while stirring for an enzymatic reaction, whereby fibrin is produced. The resulting fibrous fibrin produced by the aforementioned method is preferably separated from a reaction solution with a filter or the like, and then freeze-dried to be granulated. A method of separating and freeze-drying the thus obtained fibrin may be according to the known per se method.

The freeze-dried fibrin obtained by the aforementioned method may contain calcium. It is preferable that calcium is added when thrombin is added to fibrinogen for an enzymatic reaction. By addition of calcium, stability of fibrin is enhanced due to polymerization and crosslinking reaction of fibrin and Ca²⁺. It is preferable that Ca²⁺ to be added is 2mM or more, and in the form of a calcium salt such as CaCl₂. Fibrin is biodegradable, and by addition of calcium, a time for degradation of fibrin in a living body can be adjusted, and thus a term for inducing angiogenesis can be prolonged depending on the extent of a disease.

In addition, the freeze-dried fibrin obtained by the aforementioned method may further contain other biodegradable polymer depending on the purpose of use, site to be used, the intended retention time of fibrin in a living body or the like. Said other biodegradable polymer may be mixed with fibrin when fibrin is freeze-dried to be granulated, or when the fibrin granule is suspended in an appropriate aqueous solvent. As a biodegradable polymer, a natural polymer and a synthetic polymer are known. Examples of the natural polymer include a polysaccharide such as dextran, hyaluronic acid, chitin, chitosan, alginic acid, chondroitin sulfate, starch, pullulan, etc. or a derivative thereof, and a protein such as albumin, collagen, gelatin, etc. In the present invention, a naturally occurring polymer such as gelatin is preferable, and a natural plant polymer is particularly preferable. Examples of the synthetic polymer include polyglycolic acid, polylactic acid and polycyanoacrylate. Since these materials are absorbed later in a body and vanished, it is not necessary to consider biocompatibility. Also, since those materials have no cytotoxicity, they have no risk on a living body.

A preferable form of administration of an angiogenesis inducer containing fibrin obtained by the aforementioned method to a living body will be described below, though the form cannot be generalized and is determined by a physician, since the form varies depending on a kind of disease, the affected site, the extent of disease or the like of a patient to be administered,

The angiogenesis inducer of the present invention may be administered as a powder directly to an end site in a living body, or may be suspended in a liquid excipient such as an aqueous solvent, for example, a distilled water for injection, a physiological saline for injection, a buffer (phosphate, citrate etc.) having a pH of 5 to 8 or the like, and administered, for example, by injection or application. Alternatively, the inducer may be mixed with an appropriate excipient to be made into an ointment, a gel or a cream, and may be applied, for example, as a fibrin gel. Further, the angiogenesis inducer of the present invention containing fibrin may be formed into an appropriate shape such as a sheet, a block and a sphere, for example, a fibrin sheet, by suspending it in the aforementioned aqueous solvent and then removing the solvent therefrom, to be administered to an end site of a living body. An excipient used in these preparations is not particularly limited as far as it can be added to a medicine, but preferably it has biodegradability. Furthermore, a method for the preparation may be according to the method known in the art.

An amount of fibrin to be administered to a living body is preferably 1 to 10 mg, more preferably 1 to 5 mg, particularly preferably 2 to 3 mg per 1 cm² of a surface area of a living body to be administered to. However, since the administration amount is not necessarily limited to the above rate and cannot generalized, the amount may be determined or changed by a physician depending on the disease, condition of the site to be administered, the treating time during which fibrin is desired to stay in a living body or the like, as described above. In addition, when a dosage form of the angiogenesis inducer of the present invention is aqueous solution or ointment, the compounding ratio of an excipient and fibrin is preferably 100 to 500 µl of excipient per 4 mg of fibrin.

A method for confirming the angiogenesis-inducing effect of the angiogenesis inducer containing fibrin of the present invention is not particularly limited, but the method may be performed by administering the angiogenesis inducer of the present invention to an experimental animal (e.g. animal such as rabbit, rat or mouse), and confirming increase of arteriole at an administration site. A method for confirming arteriole formation at an administration site may be carried out, for example, by administering the angiogenesis inducer of the present invention to an experimental animal, confirming the status of the arteriole formation at the administration part with naked eyes, or collecting tissues at the administration part, fixing the tissues with formalin, staining the site with HE (hematoxylin-eosin) and investigating this. Further, blood flow rate at the administration site, an epidermal temperature or the like may be measured.

In the present invention, angiogenesis inducing effect can be confirmed by studying a skin flap take rate using a nude mouse and applying the angiogenesis inducer of the present invention on subcutaneous tissues of a part where the skin flap was peeled. More specifically, angiogenesis-inducing effect can be confirmed by opening the skin of a mouse, applying the angiogenesis inducer between the skin flap and subcutaneous tissues appeared after peeling, and measuring the blood flow rate in the subcutaneous tissues and the skin surface temperature. Alternatively, confirmation may be performed by artificially preparing an ischemic site in a living body by, for example, completely cutting an artery of a rat, administering the angiogenesis inducer to the ischemic site, and measuring blood flow rate at the site. A method of measuring blood flow rate and a skin temperature may be according to the known per se method. Examples of the method of measuring blood flow rate include a method using a realtime blood flow measurement device utilizing laser scattering, including a method using a laser Doppler device (Model ALF2100 manufactured by Advance Co. , Ltd.), and examples of the method of measuring skin temperature include a method of taking a thermography image and analyzing temperature distribution by a computer, for example, a method using a thermotracer (TH3100ME, NEC).

According to the present invention, by administering fibrin to a living body, angiogenesis can be induced, and functions of living tissues or organs suffering from dysfunction or malfunction can be regenerated. Furthermore, the effect can be promoted by administering cells and tissues to a living body together with fibrin.

For implementing regeneration therapy, a biocompatible material which is the base for tissue regeneration such as transplantation cells, or vascular growth- or cell growth-factors, and also a source for supplying oxygen and nutrients for maintaining biofunction of the transplantation cells are usually required. When these factors are introduced into a living body, and a network of these factors is formed, it becomes possible to regenerate function of living tissues or organs suffering from dysfunction or malfunction.

When the angiogenesis inducer containing fibrin of the present invention is used, fibrin can function not only as a biocompatible material but also as a source for supplying oxygen or nutrients. In other words, fibrin as a biodegradable polymer plays the both roles as the base for promoting adhesion, differentiation or morphology formation of the transplant cells, and as a source for supplying oxygen or nutrients to transplantation cells by induction of angiogenesis. Further, when the angiogenesis inducer of the present invention contains a growth factor such as bFGF, VEGF or HGF for the purpose of enhancing angiogenesis, fibrin also exerts sustained-release effect of continuously releasing a growth factor at a place of tissue regeneration.

Examples of the growth factor used in the present invention include fibroblast growth factor (FGF) including basic FGF and acidic FGF, vascular endothelial growth factor (VEGF), preferably ones derived from platelet, hepatocyte growth factor (HGF), angiopoietin including angiopoietin-1 and angiopoietin-2, platelet-derived growth factor (PDGF), insulin-like growth factor (IGF), fetal smooth muscle myosin heavy chain (SMemb), growth hormone (GH) or an analogue thereof, and other cell growth promoting factors. These growth factors may be used alone, or in combination of two or more kinds of them. These usage and doses are not particularly limited as far as they are in the known range, and cannot be generalized, since they are influenced by factors such as type of the disease of a patient to whom the angiogenesis inducer of the present invention is administered, dosage form of the angiogenesis inducer, treating term or the like. Therefore, it is preferable that usage and dose of a growth factor are determined by a physician, and the amount of growth factor to be mixed per 4 mg of fibrin is preferably in a range of about 1 ng to 100 µg, particularly preferably 1 ng to 50 µg.

Examples of the transplantation cells or transplantation tissues used in the angiogenesis inducer comprising fibrin of the present invention include an undifferentiated cell such as bone-marrow mononuclear cell, bone-marrow stromal cell, embryonic stem cell (ES cell) or the like; a differentiated cell such as keratinocyte, fibroblast, vascular endothelial cell, vascular epithelial cell, endothelial progenitor cell or the like; and a tissue comprising those differentiated cells.

A typical field of regenerative medicine is skin regeneration. When a person has a burn, or undergoes an operation of skin cancer, a wound surface remains on the skin of the patient. For curing such wound part, skin's self-regeneration ability in vivo is indispensable. By applying or injecting the angiogenesis inducer comprising fibrin of the present invention on or into a wound site, keratinocytes or fibroblasts invade into fibrin from skin tissues surrounding it, and angiogenesis is induced to promote proliferation of cells, whereby epidermal or dermal tissues are regenerated. When self-regeneration of epidermal tissues hardly occurs, by transplanting an epidermis at other part of the patient's body on a regenerated dermis, and applying the angiogenesis inducer comprising fibrin of the present invention between a regenerated dermis and a transplanted epidermis, angiogenesis is further induced and thus adhesion of an epidermis can be enhanced. In addition, when skin's self-regeneration is difficult, skin transplantation is performed for the purpose of covering protection, and adhesion of the transplanted skin can be enhanced by administering the angiogenesis inducer of the present invention between a wound site and a skin, whether a skin used for transplantation is a self skin peeled from a patient or a cultured skin cultured ex vitro. In addition, by including keratinocyte, fibroblast or the like or/and a growth factor in the angiogenesis inducer of the present invention in advance, regeneration of a self tissue and adhesiveness of a transplanted skin can be further enhanced.

There is no effective therapy for obstructive arteriosclerosis, chronic obstructive arteriosclerosis, diabetes, necropathy, and a peripheral vascular disease, typically for example, Leiner's disease and Buerger disease, and when circulation reconstruction such as vasodilation and vessel bypass is difficult, there is no choice but to amputate the lower extremity. For therapy for such the serious peripheral vascular disease, the angiogenesis inducer containing fibrin of the present invention for inducing angiogenesis and forming a new vessel is useful. By administering the angiogenesis inducer of the present invention to an affected part, a latus circulation path (bypass) due to angiogenesis is formed, and an ischemic part can be improved. For further promoting angiogenesis, vascular endothelial cells, vascular epithelial cells, endothelial progenitor cells and/or one-marrow mononuclear cells may be mixed into the angiogenesis inducer containing fibrin of the present invention in advance, and a growth factor such as bFGF, VEGF, and HGF may also be mixed.

As for heart disease such as cerebral infarction and dilated cardiomyopathy, since cardiac muscle has no regeneration ability, currently heart transplantation is the only method to treat the condition where cardiac muscle becomes contraction dysfunction due to necrosis. However, by using the angiogenesis inducer containing fibrin of the present invention, it becomes possible to regenerate cardiac muscular function. In other words, by including myocardial cells, skeletal muscle cells or smooth muscle cells in the angiogenesis inducer of the present invention, and administering this to a necrosis region of heart, regeneration of myocardial tissues occur at the necrosis region, and a vessel is induced from a circumferential healthy cardiac muscle of a host, so that the regenerated myocardial tissues could bind to the myocardial tissues of a host, resulting in regeneration of myocardial tissues which can contract synchronously.

In a brain disease such as cerebral contusion, Parkinson's disease, multiple sclerosis or cerebral infarction, transplantation of neural cells such as neural epithelial stem cells into brain has been tried in order to improve functions of a damaged brain. For treatment by nerve cell transplantation, it is required that transplanted neural stem cells take in tissue s of a host, synapse is formed, and neural circuit network is reconstructed. In order to reconstruct the neural circuit network, it is required that angiogenesis is induced around transplantation cells, and the transplantation cells are differentiated into various cells such as nerve cell, astrocyte or the like. By administering during this period the angiogenesis inducer of the present invention containing the transplantation cells into brain, angiogenesis can be simultaneously induced, and reconstruction of intracerebral neural circuit network can be promoted.

As for treatment of bone fracture or the like, a fixing equipment is directly fixed to a damaged bone in some treatment. In such cases, by using a fixing equipment together with the angiogenesis inducer of the present invention, for example, by using a fixing equipment installed with the angiogenesis inducer of the present invention, osteogenesis can be promoted and thus recovery rate is accelerated. In addition, by including osteoblasts, chondrocytes or the like in the angiogenesis inducer of the present invention, osteogenesis can be further promoted. In addition, as a method of treating a bone disease or a joint disease, artificial bone/joint replacement using an artificial bone or an artificial joint has been tried. The problem of the replacement technique is, however, how an artificial bone is fused and integrated with surrounding tissues of the patient. To solve this problem, development of an artificial bone which integrates with surrounding tissues of the patient and can grow with the surrounding tissues has been desired. In order to obtain an artificial bone having the aforementioned function, it is necessary to develop a material for an artificial bone having a network structure in which transplantations cells such as bone-marrow stem cell, osteoblast and chondrocyte can be three-dimensionally cultured, having a high biocompatibility and being absorbed into a living body in a fixed period of time is required. Since the angiogenesis inducer of the present invention contains fibrin as its essential component, it is excellent in biocompatibility and in biodegradability, and can promote proliferation of transplanted cells by angiogenesis-inducing action. Therefore, by using the angiogenesis inducer of the present invention, it becomes possible to obtain an excellent artificial joint, artificial bone or the like.

Ischemic colitis, ileus or the like known as a digestive tract disease is a serious disease accompanied with necrosis of intestinal tract tissues and intestinal tract smooth muscle cells generated by circulation disorder of an intestinal tract. In addition, an ulcerative digestive tract disease such as gastric ulcer, duodenal ulcer and ulcerative colitis is also a disease in which a smooth muscle layer is damaged due to necrosis of intestinal tract smooth muscle cells. By blending enterocyte, intestinal tract smooth muscle cells or gastric cells into the angiogenesis inducer containing fibrin of the present invention, and then administering the mixture to a necrosis region, a blood vessel is induced from circumferential healthy tissues of a host, and intestinal tract tissues or gastric tissues of the necrosis region can be regenerated.

As a method for recovering functions of an organ suffering from malfunction, a method of using an artificial organ may be exemplified. In order to realize the present method, however, an artificial organ which can be embedded in a body for a long term and can function semi-eternally is now being developed. Specifically, for the purpose of maintaining functions of such artificial organ for a long term, research and development of a bio-artificial organ, in which an artificial organ is used as a base material and culture cells are incorporated therein is being progressed. As a bio-artificial organ, there are a microcapsule type, a macrocapsule type or the like depending on its shape, and any type is equipped with a defensive system to avoid attacks of the body's immune system in the in vivo environment, for example, a defensive barrier such as an immune isolation membrane, and transplantation cells which can be a substitute for the function to be recovered, and an extracellular matrix (culture bed) to maintain functions of the transplantation cells. Moreover, the transplantation cells to be loaded in an artificial organ need to be supplied with oxygen and nutrients from blood. By combining the angiogenesis inducer comprising fibrin of the present invention and an artificial organ, it is now possible to produce a bio-artificial organ which can effectively proliferate and maintain transplantation cells loaded in the artificial organ.

Examples of the transplantation cells to be loaded in a bio-artificial organ include pancreatic islet cells, pancreatic endocrine cells, renal cells, lung epithelial cells or the like. By using those cells, abio-artificial pancreas, abio-artificial kidney, a bio-artificial lung or the like can be made. By transplanting this bio-artificial pancreas (pancreatic islet), hyperglycemia due to diabetes which is one example of endocrine and metabolism disease can be recovered to more physiologically normal state, and by transplanting a bio-artificial kidney, a physical burden of a renal failure patient who is in need of periodical blood dialysis therapy, etc. due to autoimmune deficiency can be alleviated. In addition, by transplanting a bio-artificial lung, it is possible to recover damage and breakage of lung tissues or cells, or disorder or hypofunction of lung caused by a respiratory disease such as pneumonia, fibroid lung, pulmonary pneumatosis or the like.

Further, since angiogenesis induction to an ischemic area becomes possible by the effect of the angiogenesis inducer of the present invention, subcutaneous or intramuscular transplantation of an artificial organ or a bio-artificial organ , which has been considered to be difficult, can be performed. Since subcutaneous or intramuscular transplantation can be carried out with relatively slight invasion, and recovery of the transplanted artificial organ from those area is easy, such area is thought to be an ideal transplantation site. On the other hand, a distribution density of a blood vessel is sparse in those area, and therefore proliferation and survival of cells is difficult in such area. In this regard, since angiogenesis can be induced subcutaneously or intramuscularly, and the ischemic condition of said area can be recovered by transplatation of a bio-artificial organ combined with the angiogenesis inducer of the present invention, the bio-artificial organ can be effectively function even subcutaneously or intramuscularly.

When the aforementioned cells or the like are mixed into the angiogenesis inducer of the present invention, the cells to be used may be any of cultured cells or non-cultured cells, and a method of culturing them and a method of isolating them from a living body may be according to the known method. For example, vascular endothelial cells may be endothelial cells of any vessel of artery, aorta, vein and umbilical vein, and a method of separating endothelial cells from a vessel may be, for example, a method of treating a vessel wall with a protease such as trypsin to collect freed cells. Bone-marrow mononuclear cells can be separated from a bone-marrow liquid according to a conventional method, and a bone-marrow liquid may be collected from sternum or pelvis. In addition, the separated cells may be used after culturing, if necessary. These vascular endothelial cells and bone-marrow mononuclear cells may be self-derived from a patient or a patient livestock, or may be derived from some others having transplantation compatibility, but those self-derived from a patient are preferable.

A embodiment of mixing cells into the angiogenesis inducer of the present invention is not particularly limited, but depends on factors such as the disease of a patient or the like to that the angiogenesis inducer is administered, a dosage form of the angiogenesis inducer and a treatment term, and the number of cells is generally about 1×10² to 1x10⁶ cells, particularly preferably about 1×10³ to 1x10⁵ cells per 4 mg of fibrin.

In addition, a method of mixing cells into the angiogenesis inducer of the present invention is not particularly limited, and may be carried out, for example, by uniformly suspending cells in a fibrin suspension to obtain a cell suspension, or by preparing a fibrin gel or a fibrin sheet as mentioned above and uniformly including cells therein. Accordingly, in a pharmaceutical preparation produced as mentioned above, cells mixed in the preparation are uniformly covered with fibrin and, when the preparation is administered to a living body, a vessel is effectively and uniformly regenerated. After that, fibrin which has been administered together with cells is degraded and eliminated in a living body, and the administered cells are uniformly proliferated and adhered, thereby an organ and tissues having a healthy network function can be formed.

### Examples

The present invention will be specifically explained by way of Examples, but the present invention is not at all limited to the following examples.

### [Example 1] Preparation of fibrin

500 mg of fibrinogen (manufactured by Sigma) was gradually added to 500 ml of a PBS (-) solution (pH 7.2), and this was completely dissolved while stirring with a stirrer. 125 Units of thrombin (manufactured by Sigma) was added to the resulting fibrinogen solution, and this was stirred at room temperature for 1 hour. The precipitated fibrin was collected from a solution, and washed by stirring in 500 ml of distilled water for 30 minutes. Washing was repeated three times. After washing, a moisture of fibrin was removed using a filter (5A manufactured by ADVANTEC), and the fibrin was placed into a 50 ml centrifuge tube, and freeze-stored at -80°C overnight. Frozen fibrin was dried to obtain about 280 mg of granular fibrin. Freeze-drying was performed under conditions of temperature of -40°C and overnight using FDU-830 manufactured by Tokyo Rika Kikai.

Each 4 mg of the resulting granular fibrin was subdivided into Eppendorf tubes, gas-sterilized with a gas sterilizer (Ioject SA-360 manufactured by Nishimoto Sangyo Co. , Ltd.), and stored at room temperature.

### [Test Example 1] Study of skin flap take rate using nude mouse

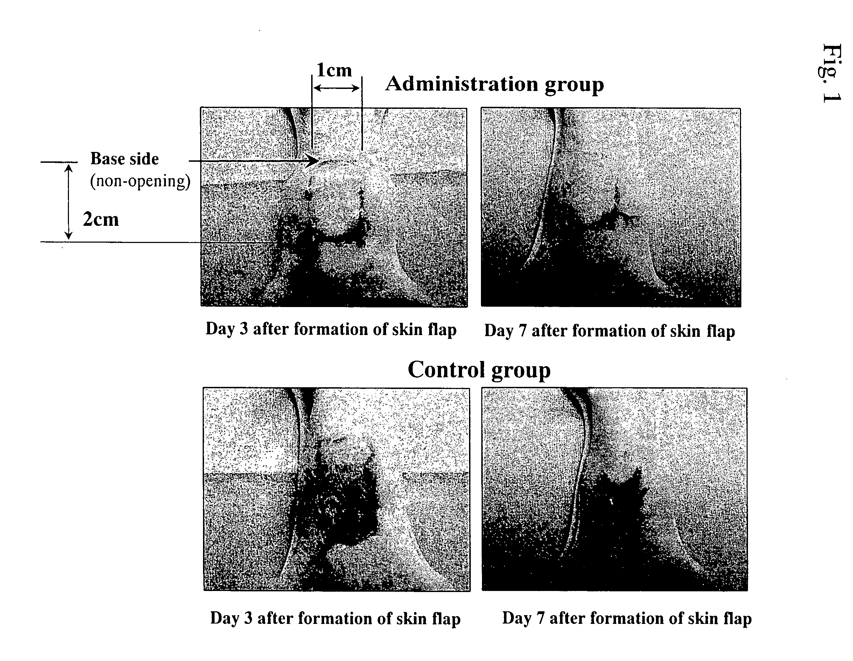

Nembutal (50 mg/kg) was intraperitoneally administered to a nude mouse (Japan SLC, Inc., BALB/C-nu), 8 to 10 week old, to anesthetize the animal, three sides of a skin on the median line part of the back were opened into a square having a traverse direction 1 cm and a length direction 2 cm without opening one side (base side: Base of Flap) in a traverse direction at a position 1 cm from an scapula, and this was peeled to prepare a skin flap (see Fig. 1).

Fibrin prepared in Example 1 was administered at 4 mg/one mouse. An administration method was performed by suspending 4 mg of fibrin in 20 µl of PBS (-) in an Eppendorf tube, and uniformly applying the solution between a skin flap and a subcutaneous tissue with a spatula. Immediately after the application, the opened part was sutured. This procedure was repeated to produce a model group (9 animals)(n=9) receiving the fibrin prepared in Example 1. As a control group, there were produced 9 mice (n=9) to which only 20 µl of PBS (-) without addition of the fibrin prepared in Example 1 had been administered. After suturing, the administration model group and the control group were returned to a rearing cage, and were reared by usually giving a solid feed and water.

### [Test Example 2] Measurement of take rate of skin flap

In an administration model group (n=9) prepared in Test Example 1 and a control group (n=9), a take rate of a skin flap on day 3 and on day 7 after skin flap formation was investigated.

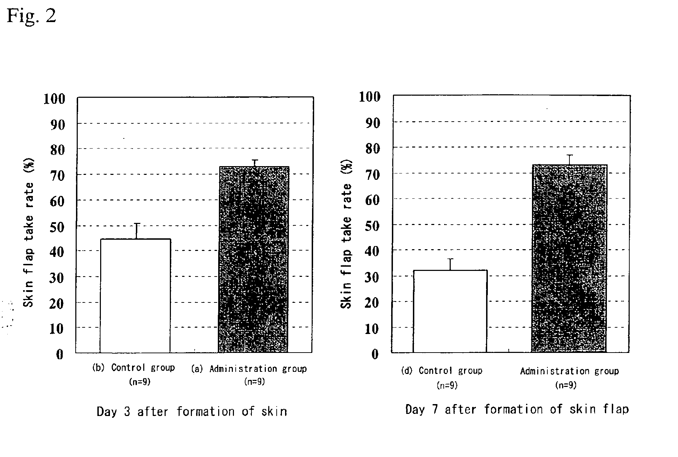

Mice of both groups on day 3 and day 7 after skin flap formation were fixed on an experimental stand, the back part was taken with a digital camera (Fig. 1), and an image was incorporated into a computer. The image was analyzed by an image processing software (Mac Aspect), and the take rate in a total skin flap was calculated. The take rate of a skin flap in both groups was obtained by subtracting the ratio of an area at a necrosis part letting an area of a total skin flap to be 100. Fig. 2 shows a take rate of a skin flap on day 3 (a) and (b) and on day 7 (c) and (d) after skin flap formation in both groups. And, t-test was performed to obtain a significant difference between both groups.

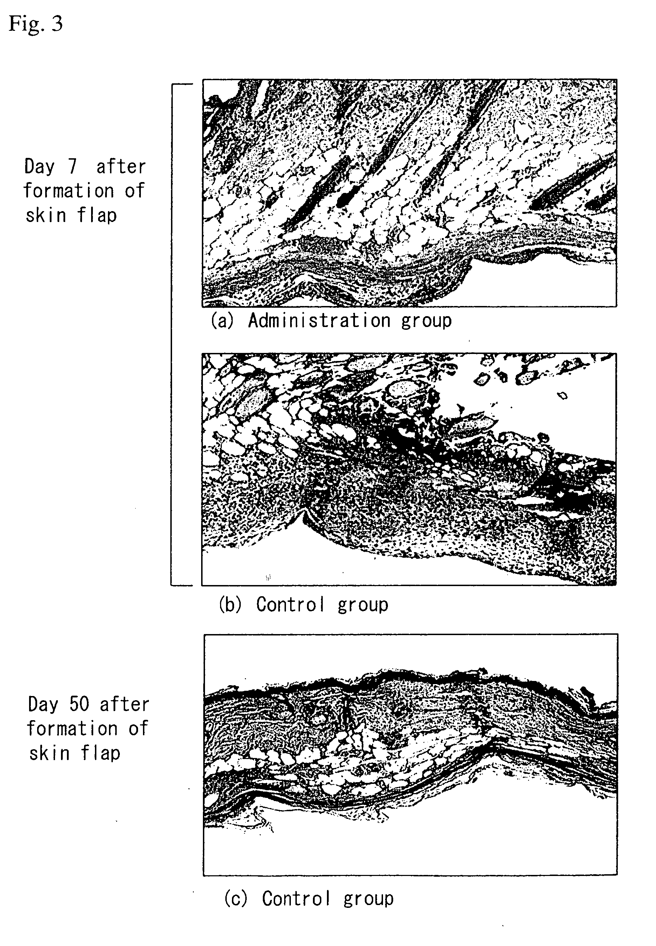

In the control group, the take rate of a skin flap was about 44.5 ± 6.07% (Mean ± SE) on day 3, and was reduced to about 32.0 ± 4.38% on day 7. As compared with the control group, in the administration model group, the take rate of a flap showed a high value of about 72.9 ± 2.54% and 73.0 ± 3.89% on day 3 and on day 7. This is because a blood vessel was newly generated in the ischemic part of a skin flap by fibrin prepared in Example 1, and adhesion between a peeled skin flap and a subcutaneous tissue was promoted. Actually, when a skin flap tissue of both groups 7 day after skin flap formation was collected, and subjected to HE staining (Fig. 3(a) (b)), formation of a more remarkable vascular network as compared with the control group was recognized in the muscle tissue, and better angiogenesis occurred, in the administration model group. As a reference, a flap tissue on day 50 after formation of a skin flap in the group of administration of fibrin prepared in Example 1 was collected, and subjected to HE staining, and results are shown in Fig. 3 (c). From this result, it was made clear that, after adhesion between a skin flap and a subcutaneous tissue, fibrin was completely degraded, and a normal tissue was formed.

### [Test Example 3] Measurement test of blood flow amount

In an administration model group (n=1) and a control group (n=1) prepared in Test Example 1, blood flow at the central part of a skin flap on day 3 and on day 7 after skin flap formation was investigated.

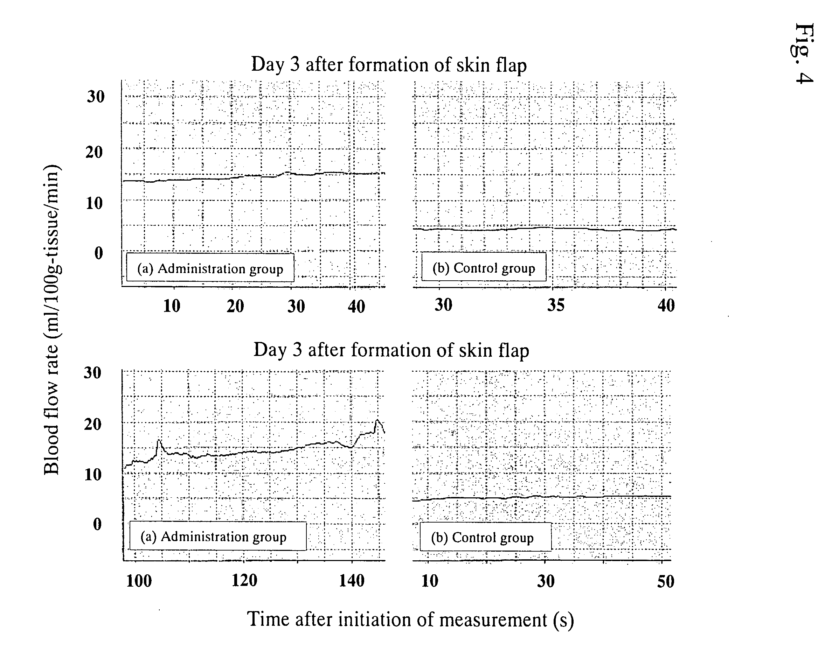

Mice of both groups were fixed on an experimental stand, a laser irradiating part of a laser Doppler apparatus (Model ALF2100, manufactured by Advance Co. , Ltd.) was put on the central part of a sutured skin flap surface at the back part, and change in blood flow amount was investigated for a constant time. Thereupon, measurement was performed by adhering an irradiation part and a mouse skin surface as much as possible. Fig. 4 (a), (b), (c) and (d) show change in blood flow amount at a time zone during which a stable blood flow amount was obtained. And, (a) and (b) show a blood flow amount (ml/100 g tissue/min) in both groups on day 3 after skin flap formation of, and (c) and (d) show a blood flow amount (ml/100 g tissue/min) in both groups on day 7 after skin flap formation.

From these results, the following was made clear. A blood flow amount of the administration model group on day 3 after skin flap formation was changed in about 14 to 16 ml/100 g tissue/min, and a blood flow amount of the control group was changed in about 4 to 5.5 ml/100 g tissue/min. In addition, on day 7 after skin flap formation, a blood flow amount of the administration model group was changed in about 11 to 20.5 ml/100 g tissue/min, and a blood flow amount of the control group was changed in about 4.5 to 5.5ml/100 g tissue/min, respectively. Therefore, in both of day 3 and day 7, a blood flow amount of the administration model group shows a more remarkably high value compared to the control group, and it was made clear that blood flow amount was elevated by administration of fibrin prepared in Example 1.

### [Test Example 4] Blood flow amount recovery test

In an administration model group (n=5) and a control group (n=4) prepared in Test Example 1, the recovery rate of a blood flow amount in a skin flap on day 1, day 3 and day 7 after skin flap formation was investigated.

Mice of both groups were fixed on an experimental stand, a line was provided in such a manner that a sutured skin flap surface on the back part (traverse direction 1 cm, length direction 2 cm square) was divided into 4 (in a length direction (length 2 cm is divided at 0.5 cm intervals), and divided into 3 in a traverse direction (traverse 1 cm is divided at about 0.33 cm intervals)). Four places of intersections of lines at a position 0.5 cm and a position 1.5 cm from a base side, and a line dividing into 3 in a traverse direction were marked, and a laser irradiating part of a laser Doppler apparatus (Model ALF2100, manufactured by Advance Co., Ltd.) was put on those four places to measure a blood flow amount (ml/100 g tissue/min). Thereupon, measurement was performed by adhering an irradiation part and a mouse skin surface as much as possible. An average of blood flow amounts at two points on a line at a position 0.5 cm from a base side was adopted as a blood flow amount at a 0.5 cm position, and an average of blood flow amounts at 2 points on a line at a position 0.5 cm from a base side was adopted as a blood flow amount at a 1.5 cm position. Letting an average of a blood flow amount obtained by measuring blood flow amounts at similar four places in advance to be 100, in mice of each of both groups before skin flap formation, a recovery rate of a blood flow amount was expressed as a ratio (%) relative to this 100. And, t-test was performed to obtain a significant difference between both groups.

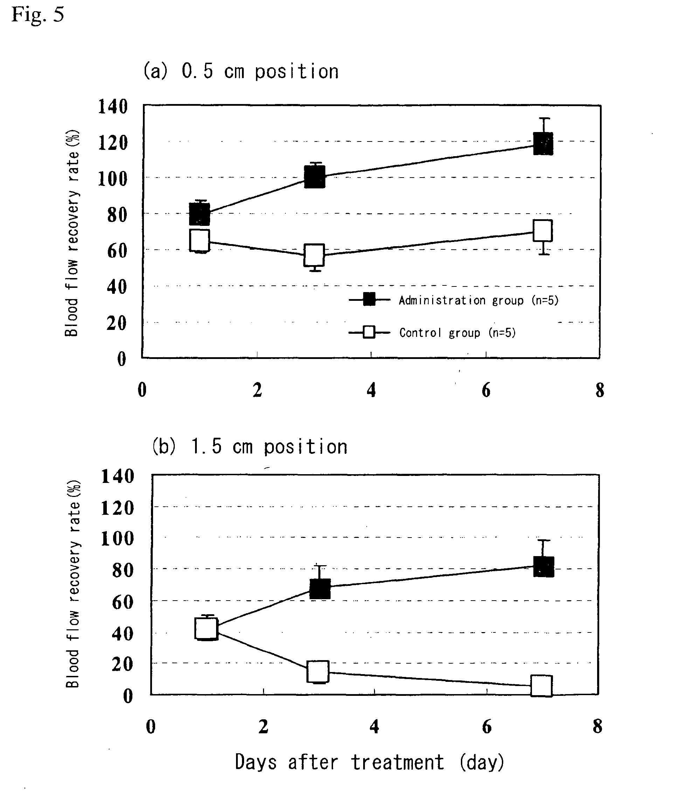

Results of a blood flow recovery rate at a 0.5 cm position and a 1.5 cm position are shown in Fig. 5 (a) and (b), respectively. In the control group, at a 0.5 cm position near a base side, recovery of blood flow amount was around 70.4 ± 13.29% (Mean ± SE) even on day 7 and, at a 1.5 cm position, only recovery of 5.23 ± 8.27% was obtained even on day 7. To the contrary, in the administration model group, a blood flow amount at a 0.5 cm position near a base side on day 3 was recovered to approximately 100% and, even at a 1.5 cm position, recovery of 81.75 ± 16.29% was seen on day 7. Therefore, it was made clear that, by administration of fibrin prepared in Example 1, remarkable recovery of blood flow amount is obtained.

Detailed experimental data are shown in Table 1.

**Table 1**

| | | Day 3 after formation of skin flap | | Day 7 after formation of skin flap | |
|---|---|---|---|---|---|
| | | Measurement position | | | |
| | | 0.5 cm | 1.5 cm | 0.5 cm | 1.5 cm |
| Control group | Blood flow amount | 7.38 ± 1.0 | 1.62 ± 0.82 | 9.25 ± 0.73 | 0.62 ± 0.61 |
| | Recovery rate (%) | 56.1 ± 7.57 | 14.4 ± 7.4 | 70.4 ± 13.29 | 5.23 ± 8.27 |
| Administration group | Blood flow amount | 15.16 ± 1.5 | 8.53 ± 0.9 | 17.41 ± 1.36 | 10.2 ± 1.08 |
| | Recovery rate (%) | 100.2 ± 8.36 | 67.95 ± 14.7 | 117.83 ± 15.17 | 81.75 ± 16.29 |
| Blood flow amount: ml /100g tissue /min | | | | | |

### [Test Example 5] Skin temperature recovery test

In an administration model group (n=5) and a control group (n=4) prepared in Test Example 1, a skin flap surface temperature on day 3 and on day 7 after skin flap formation was investigated.

Mice of both groups were fixed on an experimental stand, and a temperature of a skin surface of a region containing a sutured skin flap on the back part was taken with a thermo tracer (TH3100ME, manufactured by NEC). A mode at taking was set at a level 35°C, a sense 0.7°C, and a scan mode SCΣ4. Based on a taken image, an epidermal temperature distribution at a skin flap region was analyzed, and an average of a temperature in the region was obtained. Letting an average of values obtained by analyzing the epidermal temperature distribution at the skin flap region in advance to be 100, in mice of each of both groups before skin flap formation, a recovery rate of an epidermal temperature was expressed by the ratio (%) relative to this 100.

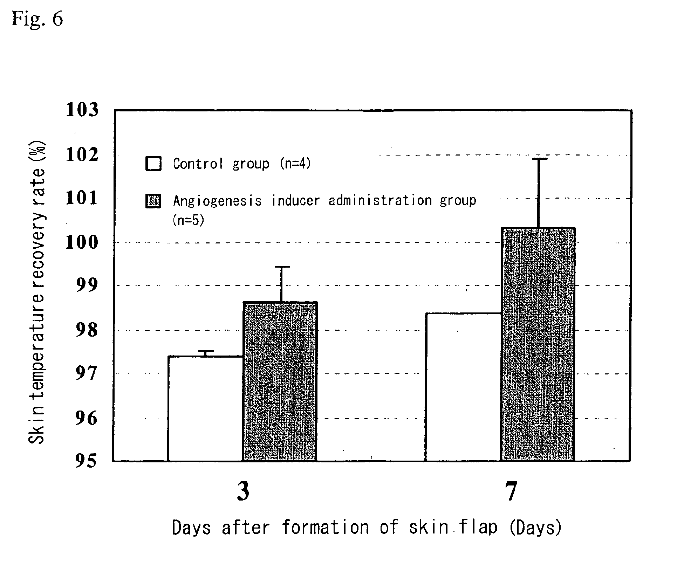

Results are shown in Fig. 6. In the administration model group, more remarkable recovery of the skin temperature was seen on day 3 and on day 7, compared to the control group. In particular, the recovery rate reached approximately 100% on day 7, and it was made clear that the skin temperature is remarkably recovered by administration of fibrin prepared in Example 1.

### [Test Example 6] Measurement test of Blood flow amount in rat ischemia model

Nembutal (50 mg/kg weight) was intraperitoneally administered to a rat (Shimizu Laboratory Supplies Co., Ltd., Kyoto), 8 to 10 week old, to anesthetize the animal. At an inner side of the rat right femoral groin, the femoral artery was completely cut to create an ischemic region at the right inferior limb (ischemia model). 8 mg of fibrin prepared in Example 1 was suspended sterile in 400 µl of PBS(-) in an Eppendorf tube, and each 100 µl of the solution was administered to four places of the right inferior limb ischemic region of an ischemia model by injection (administration model group, n=1). A blood flow amount of the light inferior limb ischemic region on day 5 after cutting of the femoral artery was measured using a laser Doppler apparatus (Model ALF2100, manufactured by Advance Co., Ltd.). A measurement method was according to Test Example 3, and blood flow amount in an ischemic region was measured. Only 400 µl of PBS (-) containing no fibrin was administered to a control group (n=1).

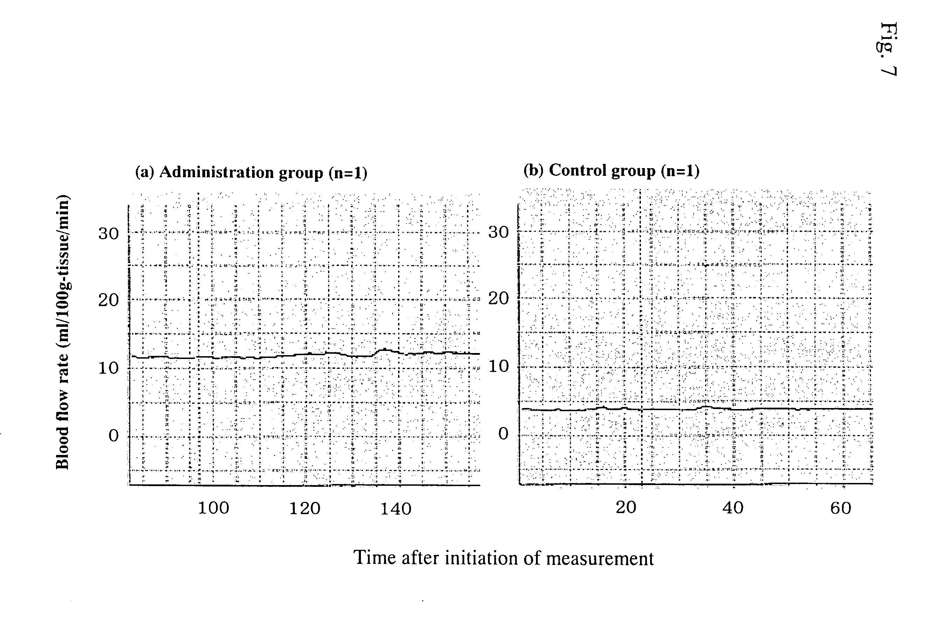

Results of the administration model group and the control group are shown in Fig. 7 (a) and (b), respectively. The blood flow amount of the administration model group was changed in about 12 to 13 ml/100 g tissue/min, and blood flow amount of the control group was changed in about 4 to 4.5ml/100 g tissue/min. The blood flow amount of the administration model group shows a remarkably higher value, compared to the control group, and it was made clear that, improvement in a blood flow amount is seen by administration of fibrin prepared in Example 1.

### INDUSTRIAL APPLICABILITY

By administering the angiogenesis inducer containing fibrin of the present invention to a living body, angiogenesis is safely and effectively induced, and thus functional regeneration of living tissues and organs suffering from dysfunction or malfunction can be achieved.

## Claims

1. An angiogenesis inducer comprising fibrin.

2. The angiogenesis inducer according to claim 1, further comprising a biodegradable polymer.

3. The angiogenesis inducer according to claim 1, further comprising a cell selected from the group consisting of bone-marrow mononuclear cell, bone-marrow stromal cell, stem cell, keratinocyte, fibroblast, myocardial cell, neural stem cell, vascular endothelial cell, endothelial progenitor cell, vascular epithelial cell, osteoblast, chondrocyte, smooth muscle cell, skeletal muscle cell, pancreatic cell, renal cell, enterocyte and stomach cell and/or a tissue comprising said selected cell.

4. The angiogenesis inducer according to claim 1, further comprising a growth factor.

5. A method of inducing angiogenesis, comprising treating a living body with fibrin.

6. A granule preparation produced by freeze-drying fibrin obtained by enzymatic degradation of fibrinogen.

7. A granule preparation produced by freeze-drying a mixture of fibrin obtained by enzymatic degradation of fibrinogen and calcium.

8. A skin graft method comprising using fibrin.

9. A method for prevention or treatment of skin disease, comprising using fibrin.

10. A method for prevention or treatment of peripheral vascular disease, comprising using fibrin.

11. A method for prevention or treatment of heart disease, comprising using fibrin.

12. A method for prevention or treatment of brain disease, comprising using fibrin.

13. A method for prevention or treatment of bone disease, comprising using fibrin.

14. A method of subcutaneously transplanting an artificial organ, comprising using fibrin.

15. A method for prevention or treatment of respiratory disease, comprising using fibrin.

16. A method for prevention or treatment of digestive organ disease, comprising using fibrin.

17. A method for prevention or treatment of endocrine and metabolism disease, comprising using fibrin.

18. A method for prevention or treatment of autoimmune disease, comprising using fibrin.

19. Use of fibrin for inducing angiogenesis.
